# EUROPÄISCHE PATENTSCHRIFT

(11) **EP 1 427 385 B1**
(45) Veröffentlichungstag und Bekanntmachung des Hinweises auf die Patenterteilung: **23.09.2015**
(21) Anmeldenummer: 02781099.3
(22) Anmeldetag: 23.09.2002
(51) Int. Cl.: A61K 39/08, A61K 38/48

(54) **ARZNEIMITTEL ZUR PROPHYLAXE UND THERAPIE VON BROMHIDROSIS**
MEDICINE FOR PREVENTING AND TREATING BROMIDROSIS
MEDICAMENT POUR LA PROPHYLAXIE ET LA THERAPIE DE BROMHIDROSE

(30) Priorität: 21.09.2001 DE 10146647
(43) Veröffentlichungstag der Anmeldung: 16.06.2004
(60) Teilanmeldung: 15179974.9
(73) Patentinhaber: ALLERGAN, INC., Irvine, CA 92612 (US)
(72) Erfinder: Heckmann, Marc, 82335 Berg (DE)
(74) Vertreter: Hoffmann Eitle
(86) Internationale Anmeldenummer: PCT/DE2002/003561
(87) Internationale Veröffentlichungsnummer: WO 2003/026602

(56) Entgegenhaltungen:
- DE-A- 19 852 981
- US-A1- 2002 086 036
- DATABASE EMBASE [Online] ELSEVIER SCIENCE PUBLISHERS, AMSTERDAM, NL EMB-2000012968, 1999 PIERARD-FRANCHIMONT C ET AL: 'How I treat.... Idiopathic hyperhidrosis' Database accession no. EMB-2000012968 & PIERARD-FRANCHIMONT C ET AL: "How I treat.... Idiopathic hyperhidrosis", REVUE MEDICALE DE LIEGE 1999 BE, vol. 54, no. 11, 1999, pages 846-850, ISSN: 0035-3663
- HECKMANN M ET AL: "AMELIORATION OF BODY ODOR AFTER INTRACUTANEOUS AXILLARY INJECTION OF BOTULINUM TOXIN A", ARCHIVES OF DERMATOLOGY, AMERICAN MEDICAL ASSOCIATION, US, vol. 139, no. 1, 1 January 2003 (2003-01-01), pages 57-59, XP009029693, ISSN: 0003-987X, DOI: 10.1001/ARCHDERM.139.1.57
- SATO K ET AL: "Biology of sweat glands and their disorders. II. Disorders of sweat gland function", JOURNAL OF THE AMERICAN ACADEMY OF DERMATOLOGY, MOSBY, INC, US, vol. 20, no. 5, 1 May 1989 (1989-05-01), pages 713-726, XP023624571, ISSN: 0190-9622, DOI: 10.1016/S0190-9622(89)70081-5 [retrieved on 1989-05-01]
- BUSHARA MAGDA AMIN ET AL: "Botulinum toxin, sweating, and body odor", NEW ENGLAND JOURNAL OF MEDICINE, vol. 347, no. 8, 22 August 2002 (2002-08-22), page 620, ISSN: 0028-4793
- NAUMANN MARKUS ET AL: "Focal hyperhidrosis. Effective treatment with intracutaneous botulinum toxin", ARCHIVES OF DERMATOLOGY, vol. 134, no. 3, March 1998 (1998-03), pages 301-304, ISSN: 0003-987X

## Beschreibung

Die vorliegende Erfindung betrifft die Verwendung von Botulinumtoxin zur Herstellung eines Arzneimittels zur Prophylaxe und/oder Therapie von Bromhidrosis sowie zur Herstellung eines kosmetischen Mittels zur Verbesserung des Körpergeruchs.

In der Kosmetik gibt es Verfahren mit fremden Stoffen (Parfums) einen angenehmen Geruch zu erzeugen, in der Medizin gibt es Verfahren zur Verminderung eines krankhaft übelriechenden Körpergeruchs (Bromhidrose). Beides ist jedoch nicht verschieden von der Möglichkeit die Wertigkeit oder Angenehmheit des körpereigenen Geruchs zu erhöhen.

Körpergeruch ist ein allgemein bekanntes und weitverbreitetes Phänomen. Es kann bei verschiedenen Individuen unter vergleichbaren Bedingungen sehr unterschiedlich stark auftreten und von den Betroffenen selbst sowie von deren Mitmenschen wiederum unterschiedlich wahrgenommen werden.

Entgegen landläufigen Vorstellungen ist der Körperschweiss, d.h. das Sekret der ekkrinen Schweissdrüsen, vollkommen geruchslos. Exzessives Schwitzen darf daher nicht mit exzessivem Körpergeruch verwechselt werden. In seiner Zusammensetzung ist Schweiss eine klare wässrige Flüssigkeit, die vorwiegend Natrium-, Kalium-, Kalzium-, Magnesium- und Chloridionen enthält, ausserdem Laktat, Harnstoff und Spuren von Aminosäuren, biogenen Aminen und Vitaminen. In Ausnahmesituationen können Medikamente über den Schweiss ausgeschieden werden, so beispielsweise Griseofulvin und Ketokonazol, wobei dies jedoch keine massgebliche Rolle für den Körpergeruch spielt. Je stärker man schwitzt, beispielsweise unter extremen Bedingungen wie in der Sauna, desto mehr wird der Schweiss verdünnt, d.h. umso wässriger wird der Schweiss. Eine übermäßige, unnatürliche Schweißproduktion unter normalen physiologischen Bedingungen ist als Krankheitsbild, der sogenannten Hyperhidrose bekannt. Übermässiges Schwitzen (Hyperhidrosis) ist also nicht die unmittelbare Ursache für Körpergeruch. Im Gegenteil: in medizinischen Lehrbüchern und Veröffentlichungen wurde explizit herausgestellt, dass Patienten die unter übermässigem Achselschweiss (Hyperhidrosis axillaris) leiden, typischerweise keinen starken Körpergeruch entwickeln [4]. Dies wird so erklärt, dass der geruchslose, in grossen Mengen ausströmende Schweiss die geruchsaktiven Substanzen auf der Haut quasi "wegspült".

Im Gegensatz zu den ekkrinen Schweissdrüsen können jedoch die sogenannten Duftdrüsen (apokrine und apo-ekkrine Drüsen) ein wahrnehmbar riechendes Sekret abgeben. Dieses Sekret sowie kann wiederum durch Bakterien auf der Hautoberfläche in übelriechende Substanzen umgewandelt werden. Den Duftdrüsen kommt daher eine ursächliche Rolle bei der Ausprägung des Körpergeruchs zu. Duftdrüsen finden sich in grosser Zahl z.B. in der Achselhöhle beim Menschen, sie werden in der Regel erst nach der Pubertät aktiv. Entsprechend ist Körpergeruch bei Erwachsenen in der Regel sehr viel stärker als bei Kindern.

Körpergeruch kann vereinfacht dargestellt also durch zwei wesentliche Faktoren verursacht werden:
1. Die durch die Duftdrüsen produzierten Aussonderungen weisen einen charakteristischen Geruch auf, der mit sehr unterschiedlicher Ausprägung von anderen wahrgenommen werden kann.
2. Die Absonderungen der Duftdrüsen, Steroidderrivate und andere körpereigene Substanzen wie z.B. Hautfette können durch die Microflora der Haut zu einer Reihe von Zersetzungprodukten abgebaut werden. Solche durch Bakterien erzeugten Zersetzungsprodukte können beispielsweise einen stechenden oder ranzigen Geruch erzeugen. Auch bestimmte Aminosäuren können zum Körpergeruch beitragen.

Insgesamt handelt es sich beim Körpergeruch also meist um eine Mischung verschiedener Komponenten, die in ihrer Zusammensetzung nicht vollständig analysiert ist und die individuell sehr unterschiedlich sein kann. Aus diesem Grund wird bei Untersuchungen zum Körpergeruch nicht nur mit biochemischen Messungen gearbeitet, sondern es werden unabhängige Testpersonen eingesetzt, die mit ihrem Geruchsorgan eine Bewertung des Körpergeruchs vornehmen und auf diese Weise auf Skalen angeben wie intensiv oder wie unangenehm ein bestimmter Geruch ist.

Körpergeruch wird bei extrem starker und unangenehmer Ausprägung als Krankheit unter dem Begriff "Bromhidrosis" definiert. Körpergeruch kann jedoch auch in einem breiten Wahrnehmungsspektrum zwischen unangenehm, ekelerregend bis hin zu angenehm oder gar betörend und stimulierend wahrgenommen werden. In der Verhaltenspsychologie wird daher bei der Wirkung von Körpergeruch nicht nur die Intensität (wie stark riecht etwas), sondern auch die emotionale Valenz (wie gut oder wie schlecht riecht etwas) bewertet

Die zur Zeit bekannten Maßnahmen zur Erlangung eines angenehmen Körpergeruchs bestehen im wesentlichen in der Anwendung von Parfums, Duftstoffen und Deodorantien zur Überdeckung des eigenen Körpergeruchs.

Therapieen zur Verminderung unangenehmen Körpergeruchs richten sich darüber hinaus in erster Linien gegen Hautbakterien und deren Zersetzungsaktivität auf der Haut. So werden bei Bromhidrosis beispielsweise empfohlen:
- Häufiges Waschen und Wechseln der Leibwäsche
- Häufige Anwendung von Seifen oder Syndets zum Abwaschen geruchsintensiver Substanzen
- Anwendung von Deodorantien
- Desinfektions - oder Hautreinigungsmittel welche die bakterielle Hautflora vermindern bzw. zerstören

Der Nachteil der vorgenannten Maßnahmen: je intensiver sie angewendet werden um so mehr führen sie zu einer Reizung der Haut und Störung der Hautbarrierefunktion, was zu unangenehmen ekzematösen Reaktionen mit Rötung und Juckreiz führen kann. Zudem haben gerade Duftstoffe in Deodorantien und Parfums ein hohe allergisierende Potenz so daß einer immunologischen Sensibilisierung Vorschub geleistet wird, die zu lebenslang bestehenden Allergien führen können.

Auch die Unterdrückung der Schweißsekretion z.B. durch Anwendung von Metallsalz-haltigen Lösungen wie Aluminium-Chlorid wird empfohlen, um eine mechanische Obstruktion der Schweißdrüsengänge und eine Eindämmung des Schweißflusses zu bewirken. Diese und ähnliche Therapien führen jedoch ebenfalls häufig zu ungewollten Hautirritationen. Darüber hinaus sind sie unter Berücksichtigung der vorgenannten gemchslosen Eigenschaften des Schweisses nicht geeignet unmittelbar auf den Körpergeruch einzuwirken, sondern können bestenfalls eine indirekte Veränderung des Hautmilieus bewerkstelligen.

Zusammenfassend lassen damit alle herkömmlichen Maßnahmen die Bedeutung der Duftdrüsen und deren Beieinflussung zur Verbesserung des Körpergeruchs ausser acht.

Die Aufgabe der Erfindung besteht daher in der Bereitstellung eines Arzneimittel zur Behandlung der Bromhidrosis und in der Bereitstellung eines kosmetischen Mittels zur Verbesserung des Körpergeruchs.

Die Aufgabe wird durch den in den Patentansprüchen definierten Gegenstand gelöst.

Die Erfindung wird durch die folgenden Figuren erläutert.

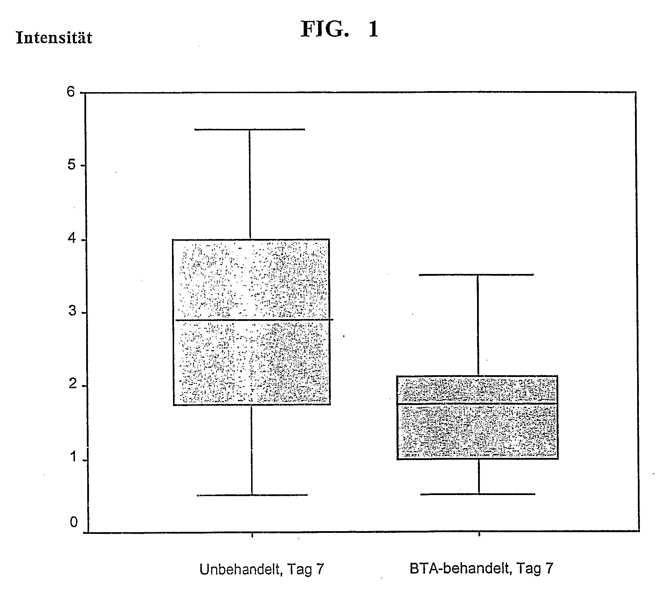

Figur 1 zeigt die Bewertung der Intensität des Körpergeruchs (0=kein Geruch wahrnehmbar, 6=maximal intensiver Geruch) nach halbseitiger Behandlung mit Botulinumtoxin in einem Diagramm. Kontroll-behandelte Achseln: n=16, Median= 2.88, SD= 1,43. Botulinumtoxin-A-behandelte Achseln n=16, Median= 1.75, SD 0,86. Signifikanz des Unterschieds (Wilcoxon-test): P = 0,02. BTA bedeutet Botulinumtoxin-A.

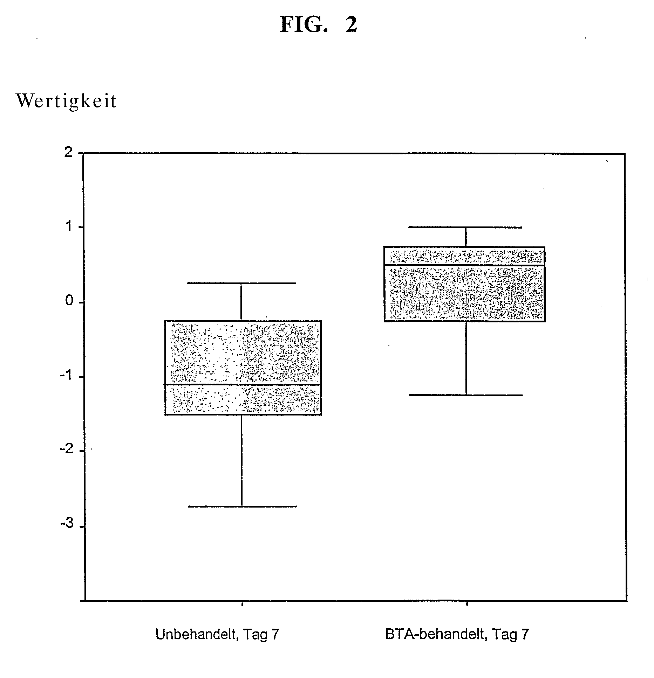

Figur 2 zeigt die Bewertung der Wertigkeit (Valenz) des Körpergeruchs (-3 = extrem unangenehm; +3 = extrem angenhm) nach halbseitiger Behandlung mit Botulinumtoxin in einem Diagramm. Kontroll-behandelte Achseln: n=16, Median= -1.13, SD= 0.89. Botulinumtoxin-A-behandelte Achseln: n=16, Median= 0.5, SD 0,75. Signifikanz des Unterschieds (Wilcoxon-test): P = 0,001. BTA bedeutet Botulinumtoxin-A.

Ein Aspekt der vorliegenden Erfindung betrifft die Verwendung von Botulinumtoxin zur Herstellung eines Arzneimittels zur Prophylaxe oder Therapie von Bromhidrosis. Ein weiterer Aspekt betrifft die Verwendung von Botulinumtoxin zur Herstellung eines kosmetischen Mittels zur Verbesserung des Körpergeruchs.

Die vorliegende Erfindung betrifft somit eine neue Methode zur Beeinflussung des Körpergeruchs z. B. in Form einer Minderung (weniger intensiv) und Verbesserung (wird als angenehmer empfunden) des Körpergeruchs. Dabei geht es nicht um eine blosse Verminderung der Schweisssekretion, da Schweiss eine geruchslose Flüssigkeit ist, sondern es geht um eine Veränderung der Geruchseigenschaften der Achselhöhle oder anderer Hautareale, die einen wahrnehmbaren und unangenehmen Geruch produzieren.

Botulinumtoxine sind eine Gruppe von hochpotenten Bakteriengiften, die durch Clostridium botulinum unter Luftabschluss erzeugt werden. Der Subtyp Botulinumtoxin-A ist seit 1989 in den USA und seit 1991 bzw. 1993 in Deutschland als medikamentöser Wirkstoff für die Behandlung ausgewählter neuromuskulärer Erkrankungen zugelassen. In Deutschland ist Botulinumtoxin-A unter dem Handelsnamen Botox® (Vertrieb durch Fa. Merz, Frankfurt; Herstellung: Allergan, Irvine Ca., USA) und dem Handelsnamen Dysport® (Vertrieb durch Fa. Ispen-pharma, Ettlingen) erhältlich. Seit 2001 ist ein weiteres Präparat mit dem Namen NeuroBloc® (Fa. Elan, München) erhältlich, welches den Subtyp Botulinumtoxin-B enthält.

Die Pharmalcologie, pharmazeutische Herstellung sowie zahlreiche klinische Anwendungen von Botulinumtoxin sind in der Fachliteratur ausführlich dargestellt [1, 2]. Die klinische Wirkung der Botulinumtoxine beruht auf einer Blockade der Acetylcholin-Freisetzung. Damit können alle Nervenendigungen die Acetylcholin als Botenstoffe benutzen blockiert werden.

Der erfolgreiche Einsatz von Botulinumtoxin-A zur Behandlung des übermässigen Schwitzens (Hyperhidrose) ist in der Fachliteratur mehrfach beschrieben [3]. Der Einfluss auf den Körpergeruch war dagegen bislang nicht bekannt. Vielmehr wurde in einer wissenschaftlichen Veröffentlichung zur Behandlung der Hyperhidrose diskutiert, daß die Injektion von Botulinumtoxin keinen Einfluss auf den Körpergeruch habe [3].

Überraschenderweise wurde jedoch vom Erfinder in klinischen Beobachtungen gefunden, daß Botulinumtoxin bei Bromhidrosis wirksam ist und auch den Körpergeruch von Gesunden verbessern kann ist. Letzteres ist keineswegs die logische Folge einer erfolgreichen Bromhidrosis-Therapie, da die Verminderung eines unangenehmen oder gar kranchaften Körpergeruchs lediglich zu einem weniger störenden oder bestenfalls neutralen Körpergeruch führen dürfte, nicht jedoch zur Erzeugung eines eigenständigen positiven Körpergeruchs.

Somit besteht ein Aspekt der vorliegenden Erfindung in der Bereitstellung einer Substanz, die dazu führt, dass der Eigengeruch des Körpers eine gesteigerte positive bzw. angenehmere Wirkung auf andere Mitmenschen entfaltet und dem Anwender somit einen Vorteil bei allen zwischenmenschlichen Beziehungen gewährt, bei denen Geruchswahrnehmung unmittelbar oder mittelbar eine Rolle spielt.

Bevorzugt wird Botulinumtoxin mittels intracutaner Injektion in die Haut eingebracht. Dies kann beispielsweise durch Verwendung einer Spritze mit feinen Injektionsnadeln bzw. Kanülen (z.B. 30 Gauge) erfolgen oder durch ein beliebiges anderes Injektionsverfahren (z.B. Hochdruck bzw. nadellose Injektion). Die Injektionen werden gleichmässig z.B. in Abstand von 0,5 bis 5 cm über das zu behandelnde Hautareal verteilt. Auch andere Injektionen z.B. subkutan oder intraepidermal sind möglich.

Auch andere Applikationsformen wie das Auftragen von Botulinumtoxin in einer geeigneten Zubereitung (z.B. Gel, Creme, Salbe, Spray) mit oder ohne Zusätzen von Stoffen, die die Penetration in die Haut fördern, sind geeignet, sofern eine transkutane Aufnahme des Wirkstoffes gewährleistet ist. Ebenso kann der Wirkstoff in einem Wasser- oder anderweitigen Lösungsmittelbad mit oder ohne Anwendung von Schwachstrom (Iontophorese) an bzw. in die Haut eingebracht werden.

Vorzugsweise wird eine gebrauchsfertige Injektionslösung von Botulinumtoxin hergestellt. Diese kann z.B. durch Auflösen einer Packungseinheit des Präparates Botox® oder des Präparates Dysport® in steriler physiologischer Kochsalzlösung (z.B. 1-10ml oder frei zu bestimmendes Volumen) zubereitet werden. Oder es wird das bereits in gelöster Form vorliegende Präparat Neurobloc® verwendet. Oder es werden beliebige Subtypen von Botulinumtoxinen (z.B. A, B, C, D, E, F, G) verwendet. Oder es werden Kombinationen mehrerer Botulinumtoxin Subtypen oder Kombinationen mit anderen Stoffen bzw. Hilfsstoffen verwendet, die zu einer der o.g. Applikationsarten geeignet sind. Die Wirkstoffkonzentration der Lösung (bestimmt in Mauseinheiten pro ml) kann den individuellen Erfordernissen und Erfahrungen entsprechend frei gewählt werden.

Vorzugsweise eignet sich die Achselhöhle zur Behandlung, es kann jedoch jede Körperregion damit behandelt werden wie beispielsweise Leistenregion, Gesäßregion, Füße. Auch jede Form der unterstützenden Vor- oder Nachbehandlung, z.B. durch Auftragen einer analgetischen Creme, Kühlung, Hautaufweichung oder einer geruchsverändernden Creme, Salbe, oder anderer Externa jeglicher Art kann mit einer der o.g. Applikationsformen kombiniert werden. Die folgenden Beispiele dienen der Erläuterung.

### Beispiel 1 - Kasuistik

Ein Patient mit starkem Körpergeruch ausgehend von der Achselhöhle erhielt pro Achselhöhle 50 Einheiten Botox® gelöst in 2 ml. NaCl verteilt auf 10 intrakutane Injektionspunkte. Nach einer Woche stellte er bei gleichen hygienischen Gewohnheiten eine deutliche Abnahme der Intensität seines Körpergeruchs fest.

### Beispiel 2 - Pilotstudie

Eine Gruppe von 16 Testpersonen wurde nach schriftlicher Aufklärung und Einwilligung untersucht. Jede Testperson wurde aufgefordert für drei Tage kein Deodorant, Parfum oder parfumierte Seife etc. zu benutzen, keine Zwiebeln, Spargel, Knoblauch zu essen und keinen intimen oder engen Kontakt mit Partnern zu haben. Am dritten Tag wurde jede Testperson aufgefordert ein weißes T-shirt (100% Baumwolle, vorgewaschen) für 24 Stunden von Mittag bis Mittag zu tragen. Danach wurden die Achselstücke der T-shirts ausgeschnitten und einzeln in luftdicht verschließbare Glasflaschen gegeben. Diese wurden anonym gekennzeichnet und den Teilnehmern dann als Geruchsprobe vorgelegt, wobei jeder Teilnehmer an allen Geruchsproben roch, ohne zu wissen von wem sie stammten. Danach erhielt jeder Teilnehmer in beiden Achselhöhlen eine Injektionsbehandlung mit zehn Einstichpunkten pro Seite. Auf der einen Seite wurden dabei 100 Einheiten Botulinumtoxin-A (Dysport®) gelöst in 2 ml isotonischer NaCl-Lösung verabreicht, auf der anderen 2 ml isotonische Kochsalzlösung. Weder Arzt noch Teilnehmer wußten welche Seite mit Wirkstoff oder mit Kontrollösung behandelt wurde (doppel-blind). Nach einer Woche wurde der T-shirt Geruchstest unter genau gleichen Bedingungen wiederholt. Nach statistischer Auswertung fand sich ein hochsignifikanter Unterschied zwischen den beiden Seiten: die mit Botulinumtoxin behandelten Achselhöhlen bzw. T-shirt-Ausschnitte rochen weniger intensiv und weniger unangenehm bzw. angenehmer als die Kontrollen.

### Beispiel 3 - Pilotstudie

56 Frauen wurden gebeten die Geruchsproben von 16 fremden Spendern zu bewerten. Die Methodik zur Herstellung der Geruchsproben war analog zu Beispiel 2, d.h. es gab von jedem Spender zwei Proben: eine von der Botulinumtoxin behandelten Achselhöhle, die andere von der unbehandelten Achselhöhle. Die Frauen wurden gebeten folgendes zu beurteilen:
1. Welche der zwei Proben riecht angenehmer (doppel-blind-Ansatz)
2. Wie ist die Geruchsqualität: (7 Punkteskala von -3 = sehr unangenehm, über 0=neutral bis zu +3 sehr angenehm)
3. Wie würden Sie den Geruch beschreiben (positive oder negative Adjektive wurden zur Wahl gestellt, z.B. blumig versus eitrig, fruchtig versus ranzig etc.)
4. Wie fühlen Sie sich wenn Sie diesen Geruch wahrnehmen (9 Wertigkeitspunkte)
5. Könnten Sie sich einen Partner mit diesem Geruch vorstellen
6. Was fällt Ihnen zu diesem Geruch ein.

Auswertung: Die paarweisen Vergleiche wurden mit Hilfe des McNemar χ² -tests für wiederholte Messungen von Nominaldaten berechnet.

In hochsignifikanter Weise wurde der Geruch der Botulinumtoxin-behandelten. Seite als angenehmer empfunden (p<0.001) und zwar sowohl im direkten Vergleich als auch gemäß der Punkteskala. Positive Adjektive wurden hochsignifikant häufiger für die Botulinumtoxin-behandelten Proben verwendet. Die Frauen fühlten sich hochsignifikant "sicherer" und "glücklicher" bei Wahrnehmung der Botulinumtoxin-behandelten Proben und sie konnten sich ebenfalls hochsignifikant häufiger vorstellen einen Partner mit dem Geruch einer Botulinumtoxin-behandelten Achselhöhle zu haben als mit dem Geruch einer unbehandelten (jeweils p< 0.001).

### LITERATURANGABEN:

1) Huang W, Foster JA, Rogachefsky AS (2000): Pharmacology of botulinum toxin. J Am Acad Dermatol 43: 249-59
2) Munchau A, Bhatia KP (2000): Uses of botulinum toxin injection in medicine today. BMJ 320: 161-5
3) Naumann M, Hofmann U, Bergmann I, Hamm H, Toyka KV, Reiners K (1998): Focal hyperhidrosis: effective treatment with intracutaneous botulinum toxin [see comments]. Arch Dermatol 134: 301-4
4) Sato K, Kang WH, Saga K, Sato KT (1989): Biology of sweat glands and their disorders. II. Disorders of sweat gland function. J Am Acad Dermatol 20: 713-726

## Patentansprüche

1. Verwendung von Botulinumtoxin zur Herstellung eines Arzneimittels zur Prophylaxe und/oder Therapie von Bromhidrosis.

2. Kosmetische Verwendung von Botulinumtoxin zur Verbesserung des allgemeinen Körpergeruchs.

3. Verwendung nach Anspruch 1 oder 2, wobei Botulinumtoxin ausgewählt ist aus Botulinumtoxin Typ A, B, C, D, E, F oder G.

## Claims

1. Use of botulinum toxin for the preparation of a medicament for the prophylaxis and/or therapy of bromhidrosis.

2. Cosmetic use of botulinum toxin for improvement of the general body odour.

3. Use according to claim 1 or 2, wherein the botulinum toxin is selected from botulinum toxin type A, B, C, D, E, F or G.

## Revendications

1. Utilisation de la toxine botulique pour la préparation d'un médicament pour la prophylaxie et/ou la thérapie de la bromhidrose.

2. Utilisation cosmétique de la toxine botulique pour l'amélioration des odeurs corporelles générales.

3. Utilisation selon la revendication 1 ou 2, dans laquelle la toxine botulique est choisie parmi la toxine botulique de type A, B, C, D, E, F ou G.
